# EUROPEAN PATENT SPECIFICATION

(11) **EP 3 236 899 B1**
(45) Date of publication and mention of the grant of the patent: **07.10.2020**
(21) Application number: 15828549.4
(22) Date of filing: 23.12.2015
(51) Int. Cl.: A61F 7/02, A61F 7/00

(54) **CONFIGURABLE CONVECTIVE DEVICE**
KONFIGURIERBARE KONVEKTIVE VORRICHTUNG
DISPOSITIF DE CONVECTION CONFIGURABLE

(30) Priority: 23.12.2014 US 201462096133 P; 02.09.2015 US 201562213498 P
(43) Date of publication of application: 01.11.2017
(60) Divisional application: 20165624.6
(73) Proprietor: 3M Innovative Properties Company, St. Paul, MN 55133-3427 (US)
(72) Inventor: MCGREGOR, Andrew J., Saint Paul, MN 55133-3427 (US)
(74) Representative: Vossius & Partner Patentanwälte Rechtsanwälte mbB
(86) International application number: PCT/US2015/000177
(87) International publication number: WO 2016/105462

(56) References cited:
- WO-A1-95/35077
- DE-U1- 9 104 142
- DE-U1- 9 104 142
- US-A- 5 824 025
- US-A- 5 947 914
- US-A1- 2005 107 856
- US-A1- 2010 211 139
- US-A1- 2014 277 307
- US-B1- 6 176 870

## Description

### Technical Field

The present disclosure relates to convective devices having pneumatic structures and accessories to be used with the convective devices, and garments with convective devices.

WO9535077 discloses a convective device with seals.

### Summary

The invention is defined in appended independent claim 1, preferred embodiments are described in the dependent claims.

### Brief Description of Drawings

The accompanying drawings are incorporated in and constitute a part of this specification and, together with the description, explain the advantages and principles of the invention. In the drawings,
Figure 1A is a top plane view of one embodiment of a convective device having separation device and optional air-guide device;
Figure 1B shows a top plane view of a reconfiguration of the embodiment illustrated in Figure 1A after two portions are partially separated;
Figure 1C is a close-up view of the convective device illustrated in Figure 1A when it is inflated and bent;
Figure 2A is a top plane view of one embodiment of a convective device having separation device and optional air-guiding element;
Figure 2B shows a top plane view of a reconfiguration of the embodiment illustrated in Figure 2A after two portions are partially separated;
Figure 2C is a close-up view of a convective device similar to the one illustrated in Figure 2A when it is inflated and bent;
Figures 3A-3L illustrate various examples of configurable convective devices;
Figures 4A-4H illustrate some examples of air-guide devices;
Figure 5A illustrates a perspective view of one embodiment of a hose clamp;
Figure 5B illustrates a side view of the hose clamp illustrated in Figure 5A;
Figure 5C illustrates another example of a hose clamp;
Figure 6A is a flattened view of a hose clamp toward the inner surface of an encircling element;
Figure 6B is a perspective view of the hose clamp illustrated in Figure 6A;
Figure 6C is a side view of the hose clamp illustrated in Figure 6A;
Figures 6D-6G illustrate some example configurations of engaging components;
Figure 7 illustrates one embodiment of a system for controlling temperature using a convective device and a hose clamp to maintain hose connection;
Figure 8 illustrates one embodiment of a gown with a convective device; and
Figure 9 illustrates an example flow diagram of using a configurable convective device.

In the drawings, like reference numerals indicate like elements. While the above-identified drawing, which may not be drawn to scale, sets forth various embodiments of the present disclosure, other embodiments are also contemplated, as noted in the Detailed Description. In all cases, this disclosure describes the presently disclosed disclosure by way of representation of exemplary embodiments and not by express limitations. It should be understood that numerous other modifications and embodiments can be devised by those skilled in the art, which fall within the scope of this disclosure.

### Detailed Description

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein. The use of numerical ranges by endpoints includes all numbers within that range (e.g. 1 to 5 includes 1, 1.5, 2, 2.75, 3, 3.80, 4, and 5) and any range within that range.

As used in this specification and the appended claims, the singular forms "a," "an," and "the" encompass embodiments having plural referents, unless the content clearly dictates otherwise. As used in this specification and the appended claims, the term "or" is generally employed in its sense including "and/or" unless the content clearly dictates otherwise.

Convective devices generally refer to a device distributing matter in gas state. For example, convective devices can receive a stream of pressurized, warmed air, inflate in response to the pressurized air, distribute the warmed air within a pneumatic structure, and emit the warmed air onto a body to accomplish such objectives as increasing comfort, reducing shivering, and treating or preventing hypothermia. In some embodiments, a convective device has a pneumatic structure that is formed by two layers, each layer including one or more sheets, and at least one of the layers is air permeable that allows air distribution. As used herein, "inflatable" refers to a structure which increases in volume when air or other gas is supplied at a pressure greater than atmospheric pressure to the interior of the structure. Typically these structures inflate at relatively low pressures such as pressures less than 100mmHg, preferably at pressures less than 50mmHg, more preferably at pressures less than 25mmHg. In some cases, the volume of the inflatable section can increase by greater than 100%.

At least some embodiments of the present disclosure direct to a convective device with features facilitating reconfiguration and transformation, for example, a portion of the convective device is bent. Typically, the pneumatic structure in the convective device is kinked or pinched off proximate to the bending area when a portion is bent. In some embodiments, the convective device includes an air-guide device disposed in the pneumatic structure adapted to direct inflation medium to reduce pressure drop of the inflation medium at the bending area. As used herein, "in" is used to describe a spatial relationship of generally in the structure including at the edge of the structure. For example, the convective device can include the air-guide device to help form one or more crease(s) when it is inflated, proximate to the air-guide device. In some cases, the convective device includes a separation device adapted to separate the pneumatic structure into two portions, where the two portions can be arranged into a different relative position from the original relative position. For example, the two portions generally extend along the same line as the original configuration; and the two portions can be partially separated and each portion can be bent about 90 degree from its original position such that the two portions become generally parallel.

At least some embodiments of the present disclosure direct to a hose clamp designed to be used with a convective device and a hose to improve air-tight connection and prevent slipping. In some embodiments, the hose clamp includes an encircling element matching the diameter of the hose and a grabbing component to facilitate user operation. In some cases, the hose clamp includes an engaging component disposed on the inner surface of the encircling element to improve gripping power of the hose clamp. The engaging component can include, for example, a plurality of engaging elements, bumps, raise-ups, or the like. In some implementations, the engaging elements are disposed in a pattern on the inner surface of the encircling element. In some cases, at least some of the engaging elements are disposed proximate to one end or both ends of the encircling element.

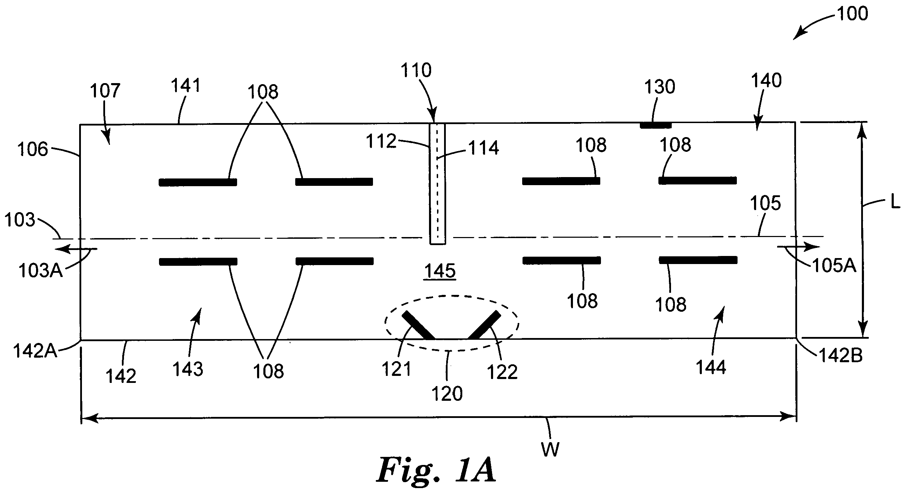

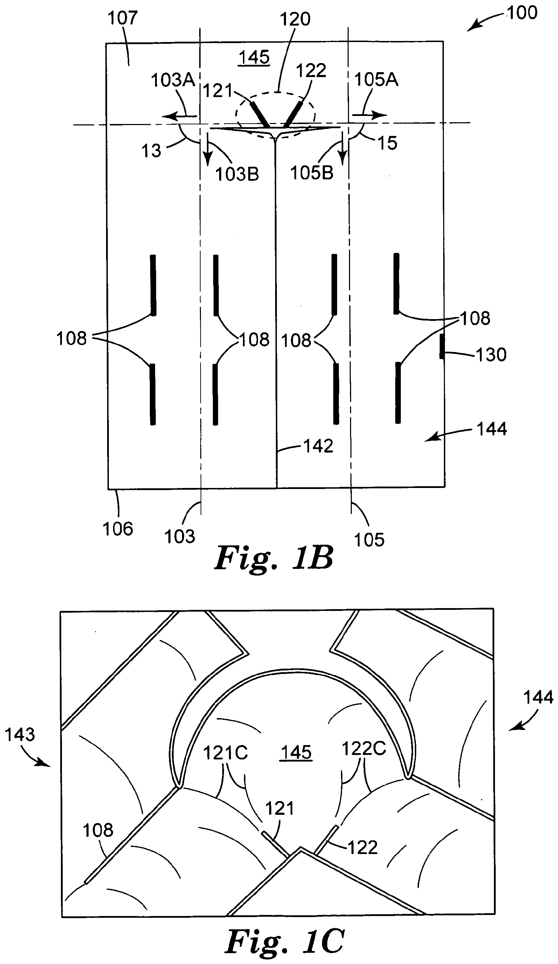

Figure 1A is a top plane view of one embodiment of a convective device 100 having separation device and optional air-guiding element; Figure 1B shows a top plane view of a reconfiguration of the embodiment illustrated in Figure 1A after two portions are partially separated; and Figure 1C is a close-up view of the convective device 100 illustrated in Figure 1A when it is inflated and bent. In this embodiment, the convective device 100 has a flexible first layer 107, a flexible second layer (not illustrated), an opening 130, an optional separation device 110, and an optional air-guide device 120. In some cases, the flexible first layer 107 has air permeable surface. The flexible second layer joining the first layer by a periphery seal 106 around a common periphery to form a pneumatic structure 140, where the pneumatic structure 140 has a first edge 141 and an opposing second edge 142. The pneumatic structure 140 has a first portion 143, a second portion 144, and an inflatable channel 145 connecting the first portion 143 and the second portion 144, where the first portion and the second portion are generally extending along a same line as illustrated in Figure 1A. In some cases, the convective device 100 can be used as an upper body blanket suitable to cover the upper body of a person with arms extending in a clinical position in its original configuration. In such cases, the convective device 100 can be rearranged to form a lower body blanket to cover the lower body of a person, for example.

In some embodiments, the convective device 100 includes at least one opening 130 into the pneumatic structure 140. The opening 130 can be in any form that allows an inflation medium source (not illustrated) to connect and provide inflation medium to inflate the pneumatic structure 140, for example, a sleeve opening at the edge as illustrated in Figure 1A. As other examples, the opening 130 can include one or more inlet ports, cuffs, ports with a rigid collar, sleeve openings at the edge, or the like.

In some embodiments, the separation device 110 is disposed proximate to the first edge 141 of the pneumatic structure 140. In some cases, the separation device includes an enclosure seal 112 extending from the first edge 141 toward the second edge 142 and a separation element 114 within the enclosure seal 112. The separation device 110 is adapted to separate the pneumatic structure into the first portion 143 and the second portion 144, such that the two portions can be rearranged and bent. The first portion 143 has a longitudinal axis 103 along a direction 103A and the second portion 144 has a longitudinal axis 105 along a direction 105A when the two portions are attached. In some cases, at least a portion of the enclosure seal 112 is generally perpendicular to the first edge. The enclosure seal 112 can be in the shape of, for example, a line, a curve, a T shape, a continuous closed shape (e.g., a rectangular shape, an oval shape, etc.), or the like. In some cases, the separation element 114 comprises at least one of a line of weakness, perforation, slit, opening, hook and loop, adhesive strip, or the like. The separation element 114 can be in a shape of, for example, line, wedge, triangular, rectangle, elliptical, curved, or the like. In some cases, the separation element 114 is a connected element to be torn open when the convective device 110 is rearranged. In some cases, the separation element includes only slim opening(s), for example, the maximum width of the opening is smaller than 2 cm. In some cases, the pneumatic structure 140 has an average length L between the first edge 141 and the second edge 142 and the length of the separation device 110 is equal to or greater than one-half of the average length L of the pneumatic structure 140.

According to the invention, the air-guide device 120 is disposed proximate to the second edge 142 of the pneumatic structure 140 and between the first portion 143 and the second portion 144, which is adapted to direct flow of inflation medium between the two portions, especially when the first portion 143 and/or the second portion 144 are bent. According to the invention, the air-guide device 120 is disposed in the inflatable channel 145 connecting the first portion 143 and the second portion 144. As used herein, "in an inflatable channel" or "within an inflatable channel" includes partially within the inflatable channel. According to the invention, the air-guide device 120 is configured to facilitate forming creases at the edge of the air-guide device when the configurable convective device 100 is inflated and at least one of the first portion and the second portion are rearranged such that part of the convective device 100 is bent (e.g., as illustrated in Figure 1C). The pneumatic structure 140 has a width W and the second edge 142 has a first end 142A and a second end 142B.

In some cases, the air-guide device 120 is disposed at a center portion of the pneumatic structure that has a starting point at a distance of 1/4 of the width from the first end 142A and an ending point at a distance of 1/4 of the width from the second end 142B. In some cases, the air-guide device 120 is disposed at a center portion of the pneumatic structure that has a starting point at a distance of 2/5 of the width from the first end 142A and an ending point at a distance of 2/5 of the width from the second end 142B. In some cases, the air-guide device 120 is disposed at the portion of the inflatable channel 145 that is closer to the second edge 142 and further away from the first edge 141. According to the invention, the air-guide device 120 comprises a guiding seal extending from the second edge 142 and toward the pneumatic structure 140. In the embodiment as illustrated, the air-guide device 120 comprises two guiding seals 121 and 122, each guiding seal extending from the second edge 142 and toward the pneumatic structure 140. According to the invention, the two guiding seals (121, 122) are directed to a different portion (the first portion 143 or the second portion 144) of the pneumatic structure 140. In some cases, the two guiding seals (121, 122) are generally perpendicular with each other.

Figure 1B illustrates how the first portion 143 and the second portion 144 of the pneumatic structure 140 can be rearranged after they are partially separated. In some cases the first portion 143 can be turned in an angle 13 between 0° and 90°, such that its longitudinal axis 103 is along a direction 103B having the angle 13 from the direction 103A. Similarly, the second portion 144 can be turned in an angle 15 between 0° and 90°, such that its longitudinal axis 105 is along a direction 105B having the angle 15 from the direction 105A. When both the first portion 143 and the second portion 144 are bent proximately 90°, the first portion 143 and the second portion 144 are arranged to be generally parallel with one another.

Figure 1C shows close-up view of the convective device 100 illustrated in Figure 1A when it is inflated and bent. The first portion 143 and the second portion 144 are rearranged in a position that the two portions are close to each other and part of the convective device 100 is bent. In the embodiment illustrated, the air-guide device 120 facilitate the formation of one or more creases 121C along the guiding seal 121 and one or more creases 122C along the guiding seal 122 such that the flow rate of inflation medium is reduced in the bending region but not greatly reduced. In some cases, the air-guide device 120 facilitate forming one or more crease(s) where the convective device 100 is bent when the convective device 100 is inflated to reduce kinking effect and air pressure drop.

Each of the first layer 107 and the second layer may include one or more sheets, where each sheet may be formed from a different material. In some implementations, the first layer 107 and/or the second layer may include an underside sheet formed from a flexible, fibrous, preferably non-woven structure composed of polymeric materials capable of bonding to an upper side sheet of a heat-sealable polymeric material. For example, the underside sheet may be a non-woven, hydroentangled polyester material and the upper side layer may include a polyolefin such as a polypropylene film which is extrusion-coated, thermally laminated, or adhesively laminated onto the polyester layer. Alternatively, the underside sheet may comprise a non-woven, paper-based material to which the upper side layer, including either a polyethylene or polypropylene film, has been glue laminated. In one embodiment, the upper side and underside sheets can be made with a stratum of absorbent tissue paper prelaminated with a layer of heat-sealable plastic. In some cases, both the first layer and the second layer can include a same polymer material.

In some embodiments, the second layer includes the upper side sheet and the underside sheet, and the first layer 107 comprises the same material as the upper side sheet of the second layer. The first layer 107 thus may include a sheet of plastic bonded to the plastic upper side of the second layer. It is preferably attached by a continuously-running web process including stations that provide an interruptible heat-sealing process. This interruptible heat sealing process can be controlled to form elongated heat seals 108 that define the inflatable channels therebetween. The seals 108 can be formed as continuous air impervious seals or discontinuous air permeable seals. The interruptible heat sealing process can be used to form the continuous seams, one of which is the seam 106 at the peripheral of the second layer and the first layer 107. In some cases, the interruptible heat sealing process can be used to form the discontinuous heat seals 108. In some cases, absorbent material can be applied to the convective device 100, for example, applied as a single material layer. The absorbent material can be bonded to the upper plastic layer by heat processing or by adhesive bonding.

In some embodiments, the convective device 100 is enabled to bathe a patient in the thermally controlled inflation medium introduced into the convective device 100, when inflated, via an air permeable layer, the first layer and/or the second layer. A layer can be air permeable using various materials or mechanical structures, for example, air-permeable materials, apertures, interstices, slits, or the like. In some implementations of an air permeable sheet with apertures, the density of apertures can vary among areas and/or inflatable sections.

In some embodiments, the first layer 107 and/or the second layer are made from a polyolefin non-woven extrusion coated, each with a coating of polypropylene on one side. In some other embodiments, the first layer 107 and/or the second layer can be poly lactic acid spunbond with polyolefin based extrusion coat. One of the first layer 107 and second layer may have holes formed by punching, slitting, or cutting to permit the flow of pressurized inflation medium from the inflated section through the layer. In some cases, the holes can be opened through both layers. In some cases, when the convective device 100 is assembled, the polypropylene-coated side of the first layer 107 is sealed to the polypropylene-coated side of the second layer at the periphery 106, and at the one or more locations 108 to form the construction. The sealing process can use various techniques, for example, ultrasonic welding, radio frequency welding, heat sealing, or the like. Alternatively, the first layer 107 and second layer may each include a laminate of polypropylene and polyolefin web with holes formed in at least one of the layers to support passage of pressurized air. In yet another embodiment, at least one of the layers can use air permeable material, for example, spunbond-meltblown-spunbond (SMS) nonwoven material, or the like.

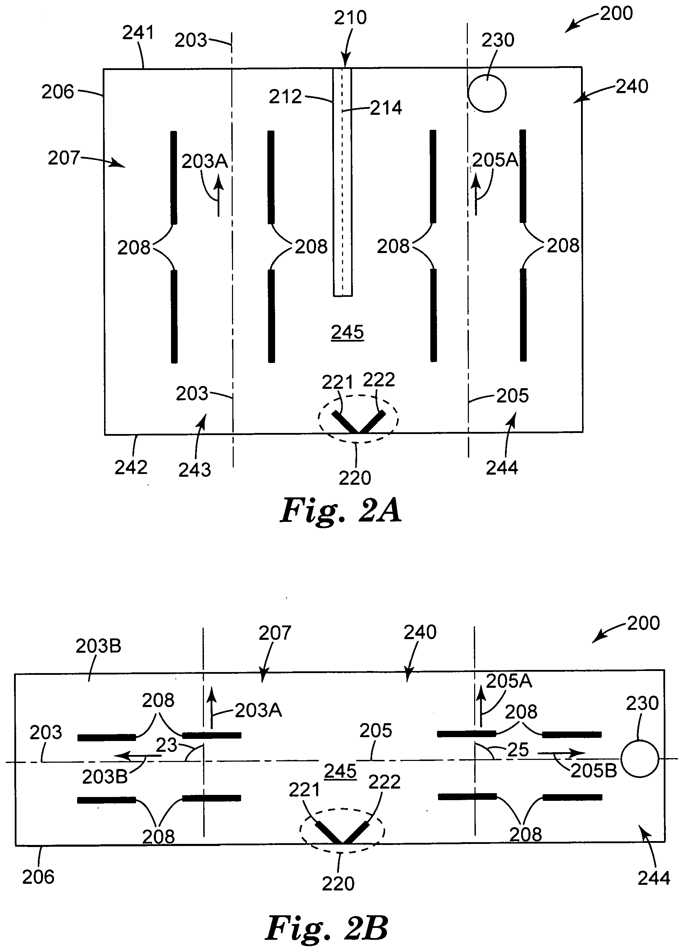

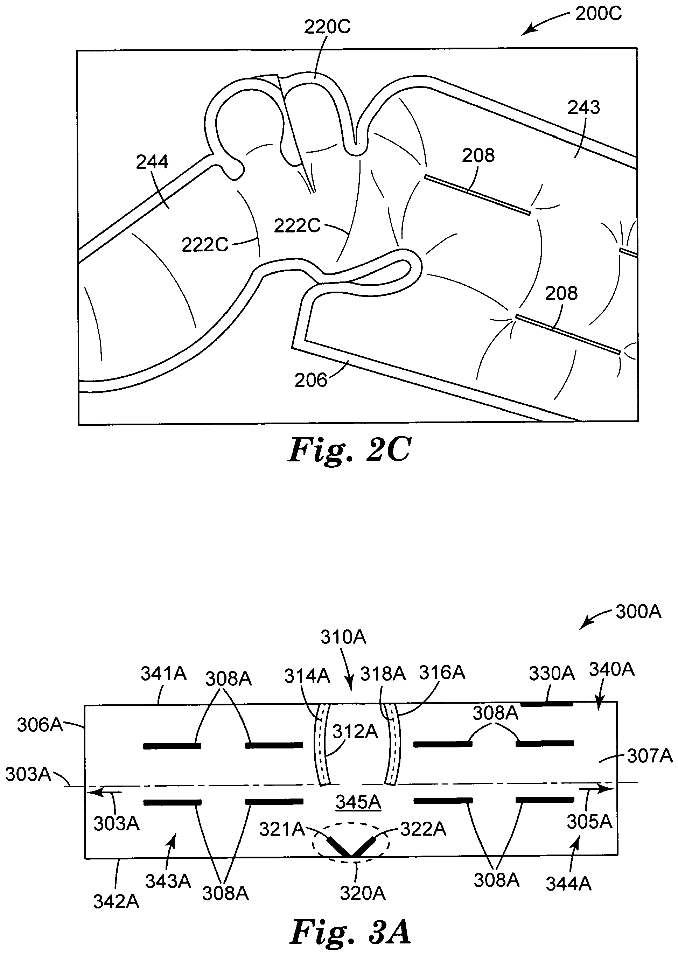

Figure 2A is a top plane view of one embodiment of a convective device 200 having separation device and optional air-guiding element; Figure 2B shows a top plane view of a reconfiguration of the embodiment illustrated in Figure 2A after two portions are partially separated; and Figure 2C is a close-up view of a convective device similar to the one illustrated in Figure 2A when it is inflated and bent. In this embodiment, the convective device 200 has a first layer 207, a flexible second layer (not illustrated), an opening 230, an optional separation device 210, and an air-guide device 220. In some cases, the flexible first layer 207 has air permeable surface. The flexible second layer joining the first layer by a seal 206 around a common periphery to form a pneumatic structure 240, where the pneumatic structure 240 has a first edge 241 and an opposing second edge 242. The pneumatic structure 240 has a first portion 243, a second portion 244, and an inflatable channel 245 connecting the first portion 243 and the second portion 244, where the first portion 243 and the second portion 244 are generally parallel with one another as illustrated in Figure 2A. In some cases, the convective device 200 is an inflatable lower body blanket, upper body blanket, or full body blanket. The components illustrated in Figures 2A-2C in can have the same or similar elements, compositions, configurations and features as the corresponding components illustrated in Figures 1A-1C.

In some embodiments, the convective device 200 includes at least one opening 230 into the pneumatic structure 240. The opening 230 can be in any form that allows an inflation medium source (not illustrated) to connect and provide inflation medium to inflate the pneumatic structure 240, for example, an inlet port as illustrated in Figure 2A. As other examples, the opening 230 can include one or more inlet ports, cuffs, sleeve openings at the edge, or the like.

In some embodiments, the separation device 210 is disposed proximate to the first edge 241 of the pneumatic structure 240. In some cases, the separation device includes an enclosure seal 212 extending from the first edge 241 toward the second edge 242 and a separation element 214 within the enclosure seal 212. The separation device 210 is adapted to separate a part of the pneumatic structure into the two portion (243, 244) such that the relative position of two portions can be rearranged. The first portion 243 has a longitudinal axis 203 along a direction 203A and the second portion 244 has a longitudinal axis 205 along a direction 205A when the two portions are attached. In some cases, at least a portion of the enclosure seal 212 is generally perpendicular to the first edge. The enclosure seal 212 can be in, for example, a line, a curve, a continuous closed shape (e.g., a rectangular shape, an oval shape, etc.), or the like. The separation element 214 comprises at least one of a line of weakness, perforation, and slit.

According to the invention, the air-guide device 220 is disposed proximate to the second edge 242 of the pneumatic structure 240 and between the first portion 243 and the second portion 244, which is adapted to direct flow of inflation medium between the two portions, especially when one or both of the two portions are bent. According to the invention, the air-guide device 220 is disposed between the first portion 243 and the second portion 244. In some cases, the air-guide device 220 is configured to facilitate forming creases at the edge of the air-guide device when the configurable convective device 200 is inflated and at least one of the first portion and the second portion are rearranged such that part of the convective device 200 is bent (e.g., as illustrated in Figure 2C). According to the invention, the air-guide device 220 comprises a guiding seal extending from the second edge 242 and toward the pneumatic structure 240. In the embodiment as illustrated, the air-guide device 220 comprises two guiding seals 221 and 222, each guiding seal extending from the second edge 242 and toward the pneumatic structure 240. According to the invention, the two guiding seals (221, 222) are directed to a different portion (the first portion 243 or the second portion 244) of the pneumatic structure 240. In some cases, the two guiding seals (221, 222) are generally perpendicular with each other.

Figure 2B illustrates how the first portion 243 and the second portion 244 of the pneumatic structure 240 can be rearranged after they are partially separated. In some cases. the first portion 243 can be turned in an angle 23 between 0° and 90°, such that its longitudinal axis 203 is along a direction 203B having the angle 23 from the direction 203A. Similarly, the second portion 244 can be turned in an angle 25 between 0° and 90°, such that its longitudinal axis 205 is along a direction 205B having the angle 25 from the direction 205A. When both the first portion 243 and the second portion 244 are turned in proximately 90° the first portion 243 and the second portion 244 are arranged to be generally extending along a same line. For example, the convective device 200 can be used as a lower body blanket to cover a person's legs and abdomen in its original configuration and be rearranged to be used as an upper body blanket to cover chest and extended arms.

Figure 2C is a close-up view of a convective device 200C similar to the one illustrated in Figure 2A when it is inflated and bent. The convective device 200C includes a periphery seal 206, seals 208 to create inflatable channels, a first portion 243 and a second portion originally arranged parallel, and an air-guide device 220C that is a wavy seal. In the embodiment illustrated, the first portion 243 and the second portion 244 are rearranged to generally extending from each other and the pneumatic structure 240 has creases 222C formed along the air-guide device 220C, such that the flow rate of inflation medium is generally uniformed within the pneumatic structure, for example, the flow rate is reduced in the bending region but not greatly reduced. In some cases, the air-guide device 220C facilitate forming one or more crease(s) 222C where the convective device 200C is bent when the convective device 200C is inflated to reduce kinking effect and pressure drop.

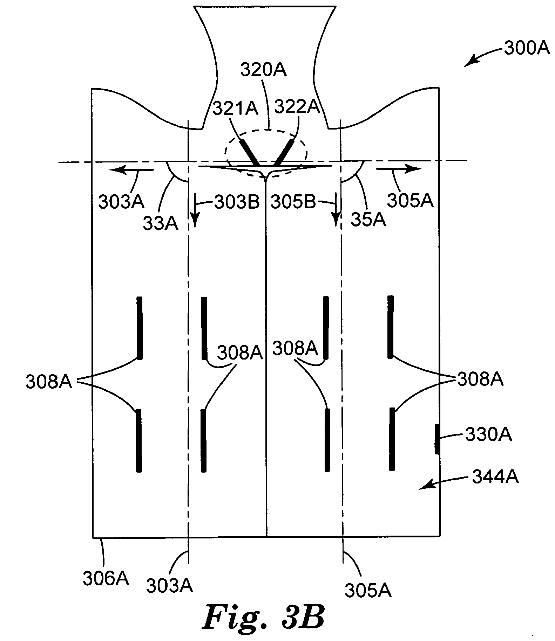

Figures 3A-3L illustrate various examples of configurable convective devices. The components in the examples illustrated in Figures 3A-3L can have the same or similar elements, compositions, configurations and features as the corresponding components illustrated in Figures 1A-2C. Figure 3A is a top plane view of one embodiment of a convective device 300A having an optional separation device and optional air-guiding element; and Figure 3B shows a top plane view of a reconfiguration of the embodiment illustrated in Figure 3A after two portions are partially separated. In this embodiment, the convective device 300A has a flexible first layer 307A, a flexible second layer (not illustrated), an opening 330A, an optional separation device 310A, and an optional air-guide device 320A. In some cases, the flexible first layer 307A has air permeable surface. The flexible second layer joining the first layer by a seal 306A around a common periphery to form a pneumatic structure 340A and seals 308A to form inflatable channels, where the pneumatic structure 340A has a first edge 341A and an opposing second edge 342A. The pneumatic structure 340A has a first portion 343A, a second portion 344A, and an inflatable channel 345A connecting the first portion 343A and the second portion 344A. In some cases, the convective device 300A can be used as an upper body blanket suitable to cover the upper body of a person with arms extending in a clinical position.

In some embodiments, the convective device 300A includes at least one opening 330A into the pneumatic structure 340A. The opening 330A can be in any form that allows an inflation medium source (not illustrated) to connect and provide inflation medium to inflate the pneumatic structure 340A, for example, a sleeve opening at the edge as illustrated in Figure 3A. As other examples, the opening 330A can include one or more inlet ports, cuffs, sleeve openings at the edge, or the like.

In some embodiments, the separation device 310A is disposed proximate to the first edge 341A of the pneumatic structure 340A. In some cases, the separation device 310A includes an enclosure seal 312A and an enclosure seal 316A extending from the first edge 341A toward the second edge 342A, where a separation element 314A is within the enclosure seal 312A and a separation element 318A within the enclosure seal 316A. The separation device 310A is adapted to partially separate the pneumatic structure into the two portions (343A, 344A) such that the relative position of the two portions can be rearranged. The first portion 343A has a longitudinal axis 302A along a direction 303A and the second portion 344A has a longitudinal axis 304A along a direction 305A when the two portions are in the original position. In the example as illustrated, the enclosure seal 312A and the enclosure seal 314A are both in curve shapes. The enclosure seal 312A and/or the enclosure seal 314A can be in, for example, a line, a curve, a continuous closed shape (e.g., a rectangular shape, an oval shape, etc.), or the like. The separation element 314A and/or the separation element 318A comprises at least one of a line of weakness, perforation, and slit. In some cases, the length of the separation device 310A is more than half of the length between the first edge 341A and the second edge 342A.

According to the invention, the air-guide device 320A is disposed proximate to the second edge 342A of the pneumatic structure 340A and adapted to direct flow of inflation medium. In some cases, the air-guide device 320A is disposed between the first portion 343A and the second portion 344A. In its original configuration, the first portion 343A is disposed along a direction 303A and the second portion 344A is disposed along a direction 305A, where both 303A and 305B are along the convective device's longitudinal axis. According to the invention, the air-guide device 320A comprises a guiding seal extending from the second edge 342A and toward the pneumatic structure 340A. In the embodiment as illustrated, the air-guide device 320A comprises two guiding seals 321A and 322A, each guiding seal extending from the second edge 342A and toward the pneumatic structure 340A. According to the invention, the two guiding seals (321A, 322A) are directed to a different portion (the first portion 343A or the second portion 344A) of the pneumatic structure 340A. In some cases, the two guiding seals (321A, 322A) are generally perpendicular with each other.

Figure 3B illustrates how the first portion 343A and the second portion 344A of the pneumatic structure 340A can be rearranged after they are partially separated. In some cases, the first portion 343A can be turned in an angle 33A between 0° and 90°, such that the first portion 343A is along a direction 303B having the angle 33A from the its original direction 303A. Similarly, the second portion 344A can be turned in an angle 35A between 0° and 90°, such that the second portion 344A is along a direction 305B having the angle 35A from its original direction 305A. When both the first portion 343A and the second portion 344A are turned in proximately 90° the first portion 343A and the second portion 344A are arranged to be generally parallel with one another.

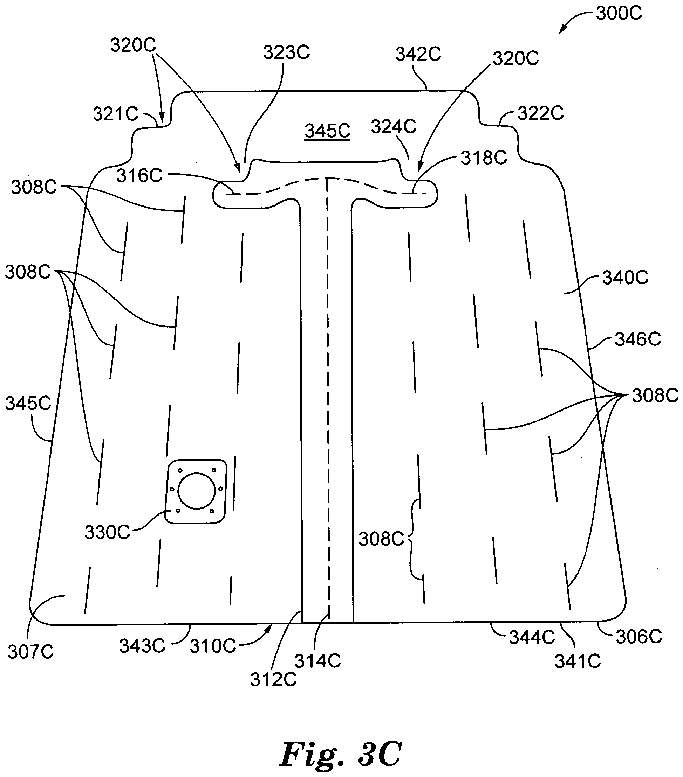

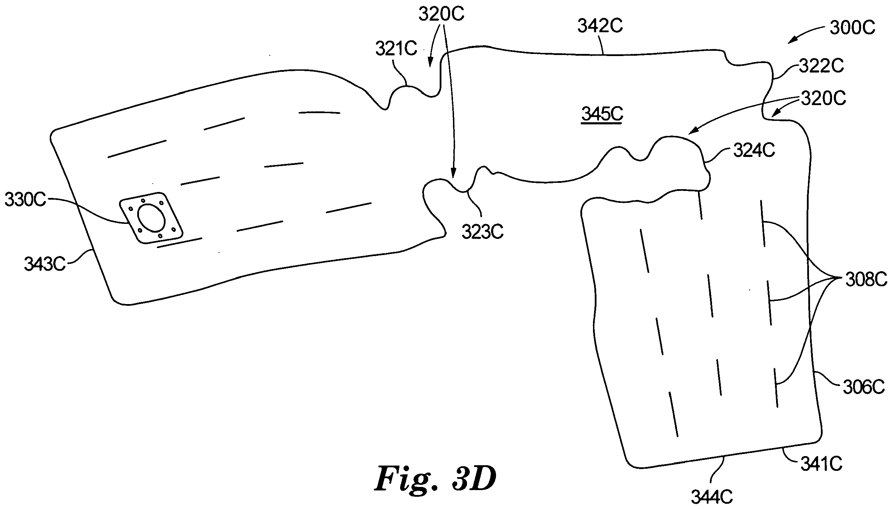

Figure 3C is a top plane view of one embodiment of a convective device 300C having an optional separation device and air-guiding element; and Figure 3D shows a top view of a reconfiguration of the example illustrated in Figure 3C after two portions are partially separated. In this embodiment, the convective device 300C has a flexible first layer 307C, a flexible second layer (not illustrated), an opening 330C, an optional separation device 310C, and an optional air-guide device 320C. In some cases, the flexible first layer 307C has air permeable surface. The flexible second layer joining the first layer by a seal 306C around a common periphery to form a pneumatic structure 340C and seals 308C to form inflatable channels, where the pneumatic structure 340C has a first edge 341C, a second edge 342C oppose to the first edge 341C, and two side edges (345C, 346C). The pneumatic structure 340C has a first portion 343C, a second portion 344C, and an inflatable channel 345C connecting the first portion 343C and the second portion 344C. In some cases, the convective device 300C is an inflatable blanket generally in a generally rectangular shape. In some cases, the convective device 300C can be used as a lower body blanket suitable to cover the lower body of a person.

In some embodiments, the convective device 300C includes at least one opening 330C into the pneumatic structure 340C. The opening 330C can be in any form that allows an inflation medium source (not illustrated) to connect and provide inflation medium to inflate the pneumatic structure 340C, for example, an inlet port with a rigid collar around the port as illustrated in Figure 3C. As other examples, the opening 330C can include one or more inlet ports, cuffs, sleeve openings at the edge, or the like.

In some embodiments, the separation device 310C is disposed proximate to the first edge 341C of the pneumatic structure 340C. In some cases, the separation device includes an enclosure seal 312C extending from the first edge 341C toward the second edge 342C, where a separation element 314C is within the enclosure seal 312C, where the separation element 314C has two extensions (316C, 318C) toward the respective side edges (345C, 346C). In the example illustrated, the extensions (316C, 318C) can be a straight line, a curved line, a slit, or the like. The separation device 310C is adapted to partially separate the pneumatic structure into the two portions (343C, 344C) such that the relative position of the two portions can be rearranged. In the example as illustrated, the enclosure seal 312C is generally in a T shape. The enclosure seal 312C can be in a shape of, for example, a line, a curve, a continuous closed shape (e.g., a rectangular shape, an oval shape, etc.), or the like. The separation element 314C comprises at least one of a line of weakness, perforation, slit, hook and loop, adhesive strip, or the like. In some cases, the longitudinal length of the separation device 310C is more than half of the length between the first edge 341C and the second edge 342C.

According to the invention, the air-guide device 320C is disposed proximate to the second edge 342C of the pneumatic structure 340C and adapted to direct flow of inflation medium. According to the invention, the air-guide device 320C is disposed within the inflatable channel 345C connecting the first portion 343C and the second portion 344C. In some examples, the air-guide device 320C comprises two guiding seals 321C and 322C, where each guiding seal (321C, 322C) is wavy connecting second edge 342C to a respective side edge (345C, 346C). In the example as illustrated, the air-guide device 320C further comprises two wavy guiding seals 323C and 324C, opposing to the two guiding seals (321C, 322C) respectively. In this example, the guiding seals (321C, 322C) are integrated with the seal 306C around the periphery. In some cases, each of the two guiding seals (321C, 322C) are proximate to a different portion (the first portion 343C or the second portion 344C) of the pneumatic structure 340C.

Figure 3D illustrates how the first portion 343C of the pneumatic structure 340C can be rearranged after they are partially separated. In some cases, the first portion 343C can be turned in an angle between 0° and 180°, or more. Similarly, while not illustrated, the second portion 344C can be turned in an angle between 0° and 180°. When both the first portion 343C and the second portion 344C are turned in proximately 90°, the first portion 343C and the second portion 344C are arranged to be generally along a same line and can be used as an upper body blanket.

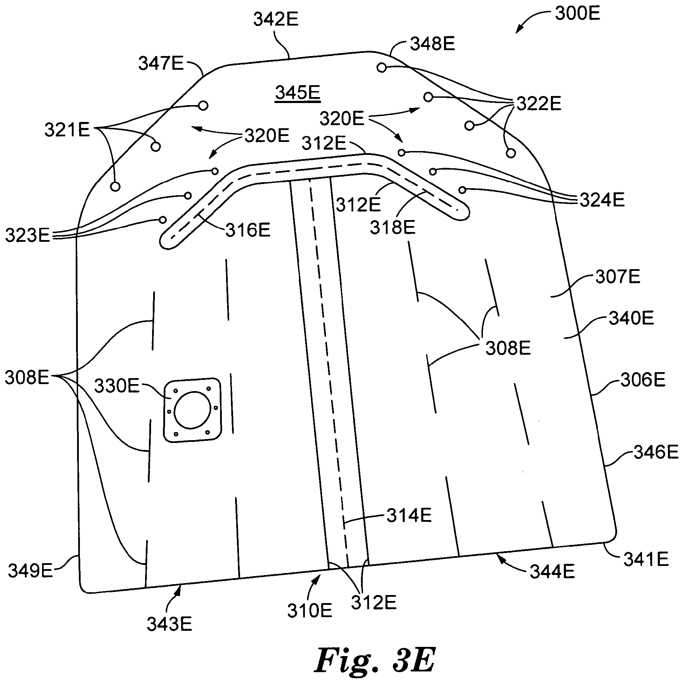

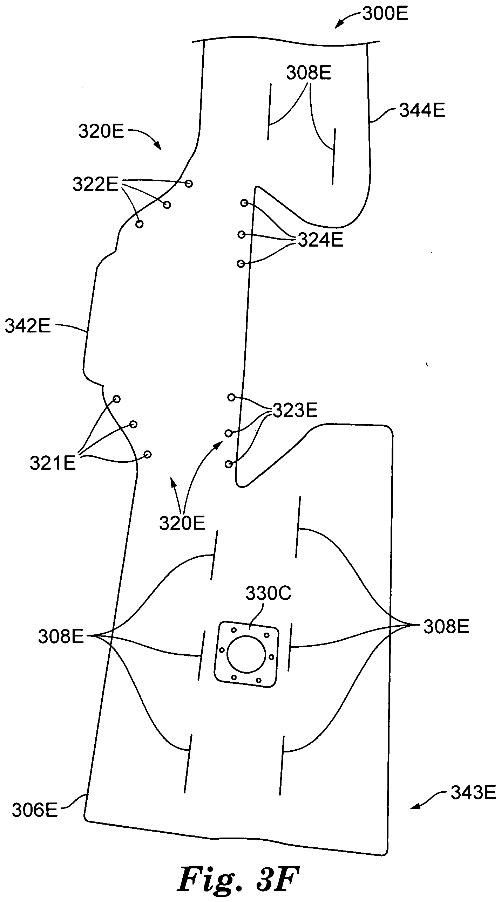

Figure 3E is a top plane view of one example of a convective device 300E having an optional separation device and optional air-guiding element; and Figure 3F shows a top view of a reconfiguration of the example illustrated in Figure 3E after two portions are partially separated. In this example, the convective device 300E has a flexible first layer 307E, a flexible second layer (not illustrated), an opening 330E, an optional separation device 310E, and an optional air-guide device 320E. In some cases, the flexible first layer 307E has air permeable surface., The flexible second layer joining the first layer by a seal 306E around a common periphery to form a pneumatic structure 340E and seals 308E to form inflatable channels, where the pneumatic structure 340E has a first edge 341E, a second edge 342E opposing to the first edge 341E, and two side edges (345E, 346E). In the example illustrated, the second edge 342E includes two slanted edges (347E, 348E). The pneumatic structure 340E has a first portion 343E, a second portion 344E, and an inflatable channel 345E connecting the first portion 343E and the second portion 344E. In some cases, the convective device 300E can be used as a lower body blanket suitable to cover the lower body of a person.

In some embodiments, the convective device 300E includes at least one opening 330E into the pneumatic structure 340E. The opening 330E can be in any form that allows an inflation medium source (not illustrated) to connect and provide inflation medium to inflate the pneumatic structure 340E, for example, an inlet port with a rigid collar around the port as illustrated in Figure 3E. As other examples, the opening 330E can include one or more inlet ports, cuffs, ports with rigid collars, sleeve openings at the edge, or the like.

In some embodiments, the separation device 310E is disposed proximate to the first edge 341E of the pneumatic structure 340E. In some cases, the separation device includes an enclosure seal 312E extending from the first edge 341E toward the second edge 342E, where the separation element 314E has two extensions (316E, 318E) toward the respective side edges (346E, 349E), where the separation element 314E with the two extensions (316E, 318E) is within the enclosure seal 312E,. In some cases, the extensions (316E, 318E) can be a straight line, a curved line, a slit, or the like. The separation device 310E is adapted to partially separate the pneumatic structure into the two portions (343E, 344E) such that the relative position of the two portions can be rearranged. In the example as illustrated, the enclosure seal 312E is generally in a T shape. The enclosure seal 312E can be in, for example, a line, a curve, a continuous closed shape (e.g., a rectangular shape, an oval shape, etc.), or the like. The separation element 314E comprises at least one of a line of weakness, perforation, slit, hook and loop, adhesive strip, or the like. In some cases, the longitudinal length of the separation device 310E is more than half of the length between the first edge 341E and the second edge 342E.

In some cases, the air-guide device 320E is disposed proximate to the second edge 342E including the slanted portions (347E, 348E) of the pneumatic structure 340E and adapted to direct flow of inflation medium. In some cases, the air-guide device 320E is disposed between the first portion 343E and the second portion 344E. In some examples, the air-guide device 320E comprises two sets of air-guide elements 321E and 322E, where each set of air-guide elements are disposed proximate to a respective slanted portion (347E, 348E) of the second edge 342E. In some cases, an air-guide element can be in any closed shape, for example, such as a circle, an oval, a square, a rectangle, a polygon, or the like. In some cases, an air-guide element can be a small seal in any shape, for example, such as a line, a curve, or the like. As used herein, a small seal refers to a seal having a length or diameter relative small, for example, less than two inches (5.08 cm). In the example illustrated, the air-guide device 320E further comprises two sets of air-guide elements 323E and 324E, opposing to the two sets of air-guide elements (321E, 322E) respectively and disposed proximately to the separation device 310E. In some cases, the two sets of air-guide elements (321E, 322E) are directed to a different portion (the first portion 343E or the second portion 344E) of the pneumatic structure 340E. In some embodiments, a set of air-guide elements can be disposed in a pattern. In some cases, a set of air-guide elements can be disposed with equal spacing. In some cases, a set of air-guide elements are disposed no more than one inch (2.54 cm) from the periphery seal 306E. In some cases, a set of air-guide elements are disposed no more than two inches (5.08 cm) from the periphery seal 306E.

Figure 3F illustrates how the first portion 343E and the second portion 344E of the pneumatic structure 340E can be rearranged after they are partially separated. In some cases, the first portion 343E can be turned in an angle between 0° and 180°. Similarly, the second portion 344E can be turned in an angle between 0° and 180°. When both the first portion 343E and the second portion 344E are turned in proximately 90° the first portion 343E and the second portion 344E are arranged to be generally in a same line.

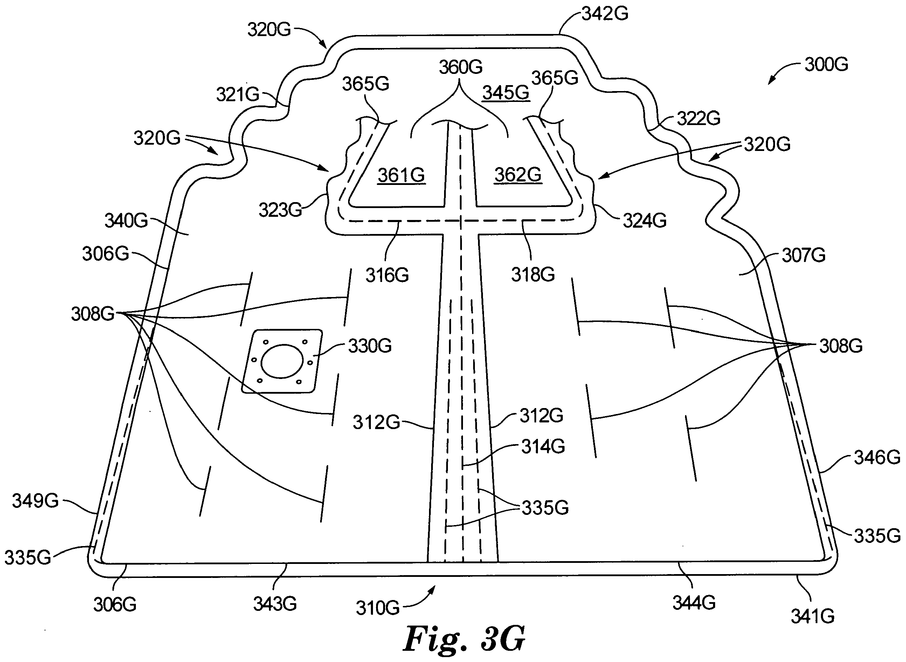

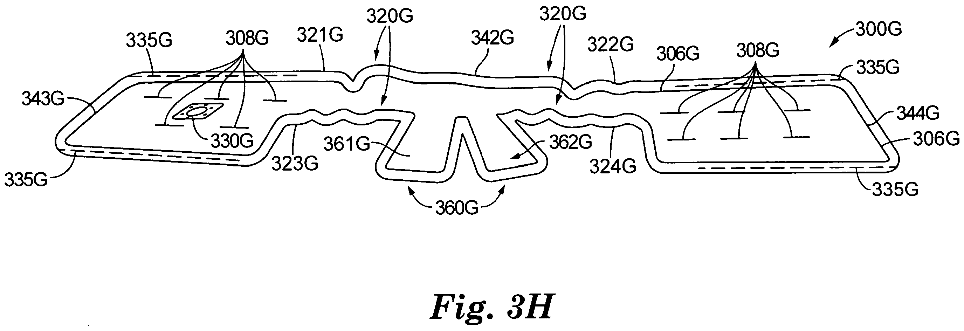

Figure 3G is a top plane view of one example of a convective device 300G having an optional separation device and optional air-guiding element; and Figure 3H shows a top view of a reconfiguration of the example illustrated in Figure 3G after two portions are partially separated. In this example, the convective device 300G has a flexible first layer 307G, a flexible second layer (not illustrated), an opening 330G, an optional separation device 310G, and an optional air-guide device 320G. In some cases, the flexible first layer 307G has air permeable surface. The flexible second layer joining the first layer by a seal 306G around a common periphery to form a pneumatic structure 340G and seals 308G to form inflatable channels, where the pneumatic structure 340G has a first edge 341G, a second edge 342G opposing to the first edge 341G, and two side edges (345G, 346G). The pneumatic structure 340G has a first portion 343G, a second portion 344G, and an inflatable channel 345G connecting the first portion 343G and the second portion 344G. In some cases, the convective device 300G can be used as a lower body blanket suitable to cover the lower body of a person.

In some embodiments, the convective device 300G includes at least one opening 330G into the pneumatic structure 340G. The opening 330G can be in any form that allows an inflation medium source (not illustrated) to connect and provide inflation medium to inflate the pneumatic structure 340G, for example, an inlet port as illustrated in Figure 3G. As other examples, the opening 330G can include one or more inlet ports, cuffs, sleeve openings at the edge, or the like.

In some embodiments, the separation device 310G is disposed proximate to the first edge 341G of the pneumatic structure 340G. In some cases, the separation device 310G includes an enclosure seal 312G extending from the first edge 341G toward the second edge 342G, where the separation element 314G has two extensions (316G, 318G) toward the respective side edges (346G, 349G), and where the separation element 314G is disposed within the enclosure seal 312G. In the example illustrated, the extensions (316G, 318G) can be a straight line, a curved line, a slit, or the like. The separation device 310G is adapted to partially separate the pneumatic structure into the two portions (343G, 344G) such that the relative position of the two portions can be rearranged. In the example as illustrated, the enclosure seal 312G is generally in a T shape. The enclosure seal 312G can be in, for example, a line, a curve, a continuous closed shape (e.g., a rectangular shape, an oval shape, etc.), or the like. The separation element 314G comprises at least one of a line of weakness, perforation, slit, or the like. In some cases, the longitudinal length of the separation device 310G is more than half of the length between the first edge 341G and the second edge 342G.

In some embodiments, the convective device 300G may include a head warmer 360G. The head warmer 360G is configured to be disposed proximate to a person's neck or head. The head warmer 360G includes two inflatable sections 361G and 362G. In the example as illustrated, the two parts (361G, 362G) can be partially separated from the convective device 300G via the separation element 365G. The separation element 365G can include at least one of a line of weakness, a slit, a line of perforation, or the like. In some cases, the head warmer 360G can have one or more wavy seal sections, which may for example, allow the head warmer to have conformity to the shape of head or neck. In some embodiments, the two parts 361G and 362G can be disposed by a person's neck when the convective device 300G is re-arranged, for example, as illustrated in Figure 3H.

In some cases, the air-guide device 320G is disposed proximate to the second edge 342G of the pneumatic structure 340G and adapted to direct flow of inflation medium. In some cases, the air-guide device 320G is disposed within the inflatable channel 345G connecting the first portion 343G and the second portion 344G. In some examples, the air-guide device 320G comprises two guiding seals 321G and 322G, where each guiding seal (321G, 322G) is a wavy seal proximate to the second edge 342G and connected to a respective side edge (346G, 349G). In the example as illustrated, the air-guide device 320G further comprises two wavy guiding seals 323G and 324G, opposing to the two guiding seals (321G, 322G) respectively and disposed proximate to the separation device 310G. In this example, the guiding seals (321G, 322G) are integrated with the seal 306G around the periphery. In some cases, the two wavy seals (321G, 322G) are directed to a different portion (the first portion 343G or the second portion 344G) of the pneumatic structure 340G.

Figure 3H illustrates how the first portion 343G and the second portion 344G of the pneumatic structure 340G can be rearranged after they are partially separated. In some cases, the first portion 343G can be turned in an angle between 0° and 180°, or an angle greater than 180°. Similarly, the second portion 344G can be turned in an angle between 0° and 180°, or an angle greater than 180°. When both the first portion 343G and the second portion 344G are turned in proximately 90°, the first portion 343G and the second portion 344G are arranged to be generally along a same line. In some embodiments that the convective device 300G includes the head warmer 360G having two parts, the two parts 361G and 362G can be disposed by a person's neck when the convective device 300G is re-arranged, for example, as illustrated in Figure 3H. In such embodiments, when the convective device 300G is inflated with heated air, the head warmer 360G can provide heated air toward the person's head area. In some embodiments, the convective device 300G may include one or more placement devices 335G that can facilitate the placement and/or configuration of the convective device 300G. The one or more placement devices 335G may include, for example, buttons, snaps, hook and loop material, tape, and/or straps, or any equivalent thereof. As illustrated in Figure 3H, the placement device 335G can be a tie strip integrally formed or defined in the convective device 300G by a line of weakness.

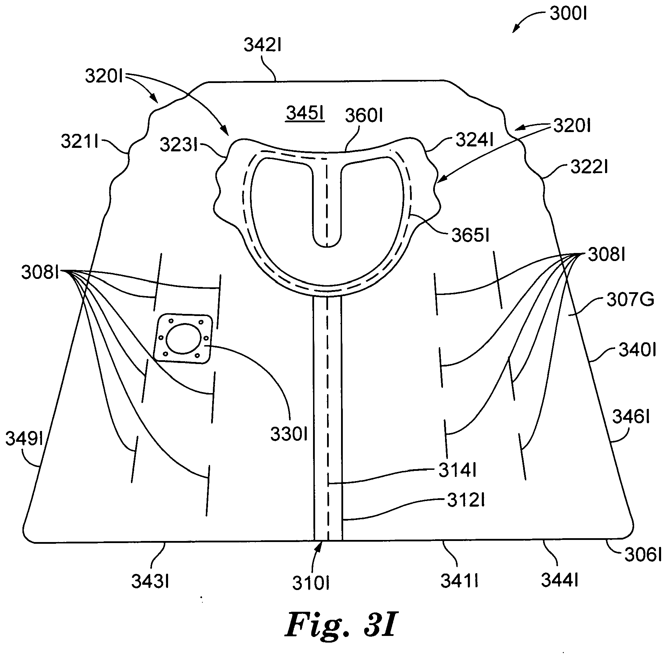

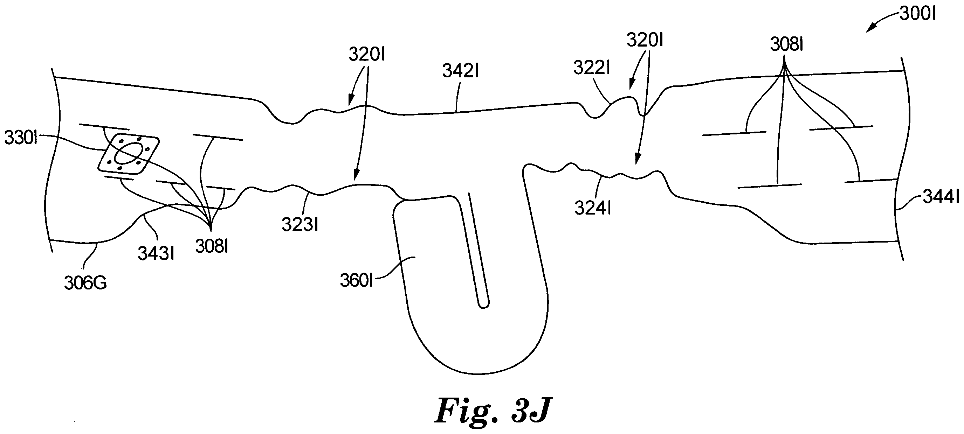

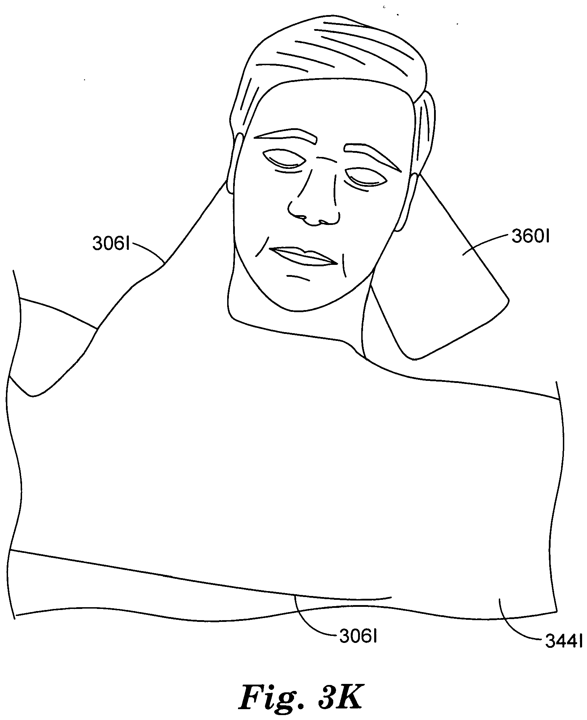

Figure 3I is a top plane view of one example of a convective device 3001 having an optional separation device and optional head warmer; Figure 3J shows a top view of a reconfiguration of the example illustrated in Figure 3I after head warmer is partially separated; and Figure 3K illustrates how the head warmer can be used. In this example, the convective device 300I has a flexible first layer 3071, a flexible second layer (not illustrated), an opening 3301, an optional separation device 3101, and an optional air-guide device 3201. In some cases, the flexible first layer 3071 has air permeable surface. The flexible second layer joining the first layer by a seal 3061 around a common periphery to form a pneumatic structure 3401 and seals 3081 to form inflatable channels, where the pneumatic structure 3401 has a first edge 341I, a second edge 3421 opposing to the first edge 3411, and two side edges (3451, 3461). The pneumatic structure 3401 has a first portion 3431, a second portion 3441, and an inflatable channel 3451 connecting the first portion 3431 and the second portion 3441. In some cases, the convective device 300I can be used as a lower body blanket suitable to cover the lower body of a person.

In some embodiments, the convective device 3001 includes at least one opening 3301 into the pneumatic structure 3401. The opening 3301 can be in any form that allows an inflation medium source (not illustrated) to connect and provide inflation medium to inflate the pneumatic structure 3401, for example, an inlet port as illustrated in Figure 3I. As other examples, the opening 3301 can include one or more inlet ports, cuffs, ports having stiff collars, sleeve openings at the edge, or the like.

In some embodiments, the separation device 3101 is disposed proximate to the first edge 3411 of the pneumatic structure 3401. In some cases, the separation device includes an enclosure seal 3121 extending from the first edge 341I toward the second edge 3421, where a separation element 3141 is within the enclosure seal 3121. The separation device 3101 is adapted to partially separate the pneumatic structure into the two portions (3431, 3441) such that the relative position of the two portions can be rearranged. The enclosure seal 3121 can be in, for example, a line, a curve, a continuous closed shape (e.g., a rectangular shape, an oval shape, etc.), or the like. The separation element 3141 comprises at least one of a line of weakness, perforation, slit, or the like. In some cases, the longitudinal length of the separation device 3101 is more than half of the length between the first edge 341I and the second edge 3421.

In some cases, the air-guide device 3201 is disposed proximate to the second edge 3421 of the pneumatic structure 3401 and adapted to direct flow of inflation medium. In some cases, the air-guide device 3201 is disposed within the inflatable channel 3451 connecting the first portion 3431 and the second portion 3441. In some examples, the air-guide device 3201 comprises two guiding seals 321I and 3221, where each guiding seal (3211, 3221) is a wavy guiding seal proximate to the second edge 3421 and connected to a respective side edge (3461, 3491). In the example illustrated, the air-guide device 320I further comprises two wavy guiding seals 3231 and 3241, opposing to the two guiding seals (3211, 3221) respectively and disposed proximate to the separation device 3101. In this example, the guiding seals (321I, 322I) are integrated with the seal 3061 around the periphery. In some cases, the two wavy seals (321I, 322I) are directed to a different portion (the first portion 3431 or the second portion 3441) of the pneumatic structure 3401.

Figure 3J illustrates how the first portion 3431 and the second portion 3441 of the pneumatic structure 3401 can be rearranged after they are partially separated. In some cases, the first portion 3431 can be turned in an angle between 0° and 180°, or an angle greater than 180°. Similarly, the second portion 3441 can be turned in an angle between 0° and 180°, or an angle greater than 180°. When both the first portion 3431 and the second portion 3441 are turned in proximately 90° the first portion 3431 and the second portion 3441 are arranged to be generally in a same line.

In some embodiments, the convective device 3001 includes a head warmer 3601 that is generally in a U shape. The head warmer 3601 can be partially separated from the convective device 3001 via a separation element 365I. The separation element 3651 can include at least one of a line of weakness, a slit, a line of perforation, or the like. In some embodiments, the head warmer 3601 can be disposed by a person's neck or head when the convective device 3001 is re-arranged, for example, as illustrated in Figure 3K. In some cases, the head warmer 3601 can have one or more wavy seal sections, which may for example, allow the head warmer to have conformity to the shape of head or neck. In some cases, the head warmer 3601 may include a portion that can be completely separated from the convective device by the separation element 365I.

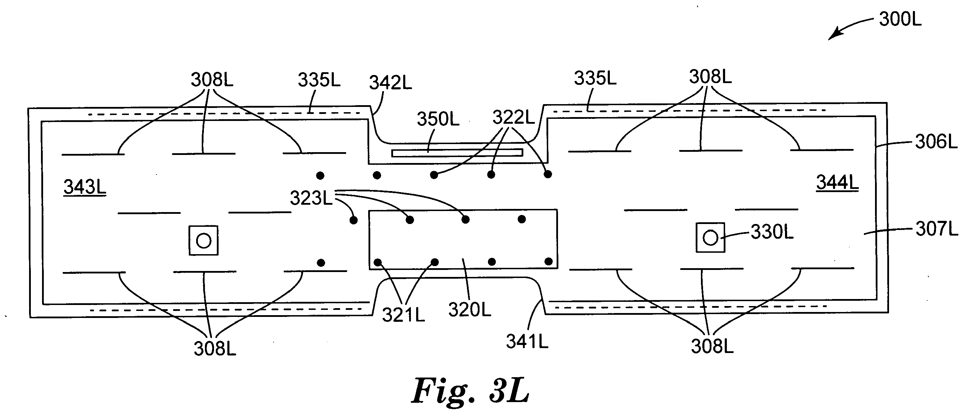

Figure 3L is a top plane view of one example of a convective device 300L having an optional air-guiding element. In this embodiment, the convective device 300L has a flexible first layer 307L, a flexible second layer (not illustrated), an opening 330L, and an optional air-guide device 320L. In some cases, the flexible first layer 307L has air permeable surface. The flexible second layer joining the first layer by a seal 306L around a common periphery to form a pneumatic structure 340L and seals 308L to form inflatable channels, where the pneumatic structure 340L has a first edge 341L, a second edge 342L opposing to the first edge 341L. The pneumatic structure 340L has a first portion 343L, a second portion 344L, and an inflatable channel 345L connecting the first portion 343L and the second portion 344L. In some cases, the convective device 300L can be used as an inflatable upper body blanket suitable to cover the upper body of a person.

In some embodiments, the convective device 300L includes at least one opening 330L into the pneumatic structure 340L. The opening 330L can be in any form that allows an inflation medium source (not illustrated) to connect and provide inflation medium to inflate the pneumatic structure 340L, for example, an inlet port with a rigid collar around the port as illustrated in Figure 3L. As other examples, the opening 330L can include one or more inlet ports, cuffs, ports with rigid collars, sleeve openings at the edge, or the like.

In some examples, the air-guide device 320L includes two sets of air-guide elements 321L and 322L, where each set of air-guide elements are disposed proximate to a respective edge (341L, 342L). In some cases, the air-guide device 320L includes one or more sets of air-guide elements disposed in a pattern. In some cases, a set of air-guide elements can be disposed with equal spacing. In some cases, a set of air-guide elements are disposed no more than one inch (2.54 cm) from the periphery seal 306L. In some cases, a set of air-guide elements are disposed no more than two inches (5.08 cm) from the periphery seal 306L. In some cases, the air-guide device 320L includes three sets of air-guide elements 321L, 322L, and 323L disposed in a staggered pattern.

The first portion 343L and the second portion 344L of the pneumatic structure 340L can be rearranged. In some cases, the first portion 343L can be turned in an angle between 0° and 180°. Similarly, the second portion 344L can be turned in an angle between 0° and 180°. When both the first portion 343L and the second portion 344L are turned in proximately 90° the first portion 343L and the second portion 344L are arranged to be generally in a same line. When the first portion 341L and/or the second portion 342L is rearranged, the air-guide device 320L can facilitate forming one or more crease(s) where the convective device 300L is bent when the convective device 300L is inflated to reduce kinking effect and air pressure drop.

In some embodiments, the convective device 300L may include one or more placement devices (335L, 350L) that can facilitate the placement and/or configuration of the convective device 300L. The one or more placement devices may include, for example, adhesive patches or strips, buttons, snaps, hook and loop material, tape, and/or straps, or any equivalent thereof. As illustrated in Figure 3L, the placement device 335L can be a tie strip integrally formed or defined in the convective device 300L by a line of weakness. As another example, the placement device 340L can be an adhesive strip disposed along an edge (e.g., edge 342L) of the convective device 300L.

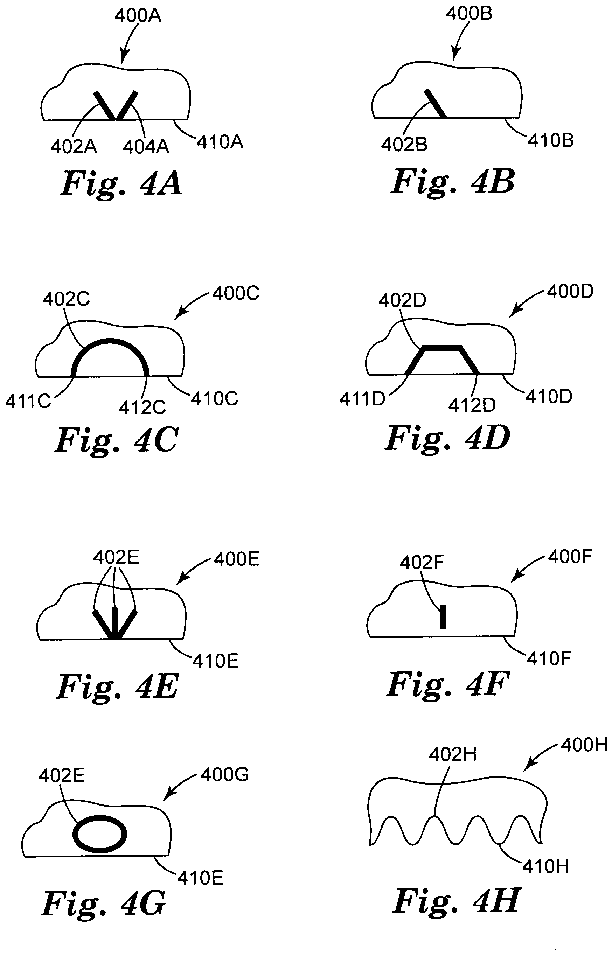

Figures 4A-4H illustrate some examples of air-guide devices/air-guide elements, figures 4A and 4E are embodiments according to the invention. Figure 4A illustrates an air-guide device or air-guide element 400A including two guiding seals 402A and 404A, where both seals are extending from a periphery seal 410A. Figure 4B illustrates an air-guide device or air-guide element 400B including one guiding seal 402B extending from a periphery seal 410B. Figure 4C illustrates an air-guide device or air-guide element 400C including a continuous seal 402C, in a curve shape, starting from a first position 411C on a periphery seal 410C and ending at a second position 412C on the periphery seal 410C different from the first position 411C. When the convective device is inflated, the air-guide device 400C can facilitate forming a number of creases proximate to the air-guide device 400C in the convective device where the convective device is bent. Figure 4D illustrates an air-guide device or air-guide element 400D including a continuous seal 402D starting from a first position 411D on a periphery seal 410D and ending at a second position 412D on the periphery seal 410D different from the first position 411D.

Figure 4E illustrates an air-guide device or air-guide element 400E including three guiding seals 402E extending from a periphery seal 410E. Figure 4F illustrates an air-guide device or air-guide element 400F including one seal 402F disposed proximate to but not touching a periphery seal 410F. Figure 4G illustrates an air-guide device or air-guide element 400G including one continuous seal 402G in a closed shape or a closed shape seal 402G disposed proximate to but not touching a periphery seal 410G. The seal 402G can be in any closed shapes, for example, such as circle, oval, square, rectangle, polygon, or the like. In some cases, the seal 402G is no more than one inch (2.54 cm) from the periphery seal 410G. In some cases, the seal 402G is no more than two inches (5.08 cm) from the periphery seal 410G.

Figure 4H illustrates an example of air-guide device 400H that is an integrated part of or proximate to a periphery seal 410H. The air-guide device 400H includes a zigzag portion 402H between first and second portions of the pneumatic structure as illustrated in Figures 1A, 2A and 3A, for example, such that the zigzag portion 402H is adapted to facilitate the generation of a number of distributed creases and direct inflation medium to reduce pressure drop of the inflation medium at the bending area when the configurable convective device is inflated and at least one of the first portion and the second portion are rearranged such that part of the convective device is bent. In some cases, the zigzag portion 402H is integrated with the periphery seal 410H. In some cases, the entire periphery seal can be zigzagged. In some cases, the zigzag portion 402H is in a wavy shape. In some cases, the zigzag portion is a in a curve shape, square saw-tooth, triangular saw-tooth, or similar shape, or combination of shapes.

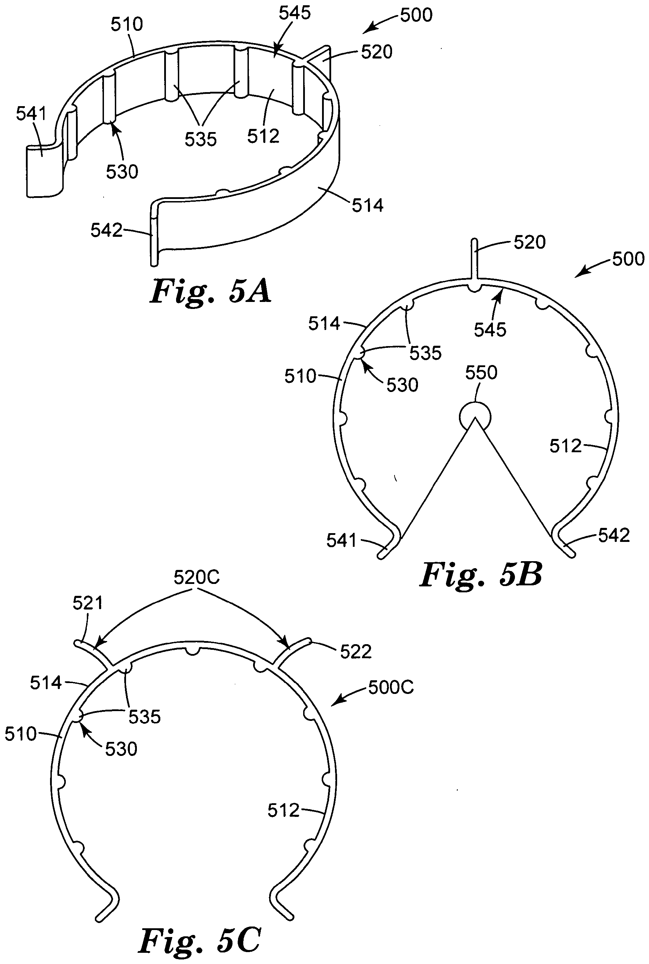

In some cases of using a convective device, a hose clamp may be used to maintain adequately air-tight connection between the hose and the convective device. Embodiments of the hose clamp are not part of the present invention. Figure 5A illustrates a perspective view of one embodiment of a hose clamp 500; and Figure 5B illustrates a side view of the hose clamp 500. In the embodiment illustrated, the hose clamp 500 includes an encircling element 510, an optional grabbing component 520 extending from the encircling element, and an optional engaging component 530 disposed on or integrated with the encircling element. The encircling element 510 includes having an inner surface 512 and an opposing outer surface 514. In some cases, the central angle 550 of the encircling element 510 is greater than 180 degree. In some cases, the central angle 550 of the encircling element 510 is smaller than 360 degree.

In some embodiments, the engaging component 530 includes a plurality of engaging elements 535. In some implementations, the engaging component 530 includes a pattern of engaging elements 535, for example, a pattern of a line, a pattern of a wave, a pattern of higher density proximate to the end, or the like. The encircling element 510 has a first end 541, a second end 542, and a middle portion 545. In some cases, the encircling element 510 can be semi-rigid or rigid. The encircling element 510 can include materials, for example, polycarbonate, polyethylene, nylon, acrylonitrile butadiene styrene (ABS), polypropylene, polyvinyl chloride (PVC), and/or the like. In some cases, the grabbing component 520 and the engaging component 530 can include the same materials as the encircling element 510. In some other cases, the grabbing component 520 and the engaging component 530 can include different materials as the encircling element 510. In some cases, the engaging component can have a material the same as or different from the material used for the encircling element 510. In some cases, the engaging component 530 can use soft materials, for example, urethane, thermoplastic materials, thermoplastic elastomers (TPE), or the like. The engaging elements 535 can have any shapes, for example, cylinder, half sphere, prism, hexagonal prism, trapezoidal prism, cube, cuboid, cone, pyramid, or the like.

Figure 5C illustrates a front view of another embodiment of a hose clamp 500C. The hose clamp 500C includes an encircling element 510, an optional grabbing component 520C extending from the encircling element, and an optional engaging component 530 disposed on or integrated with the encircling element. Components with same labels can have same or similar configurations, compositions, functionality and/or relationships as the corresponding components in Figures 5A and 5B. In the embodiment illustrated, the grabbing component 520C includes two elements 521 and 522.

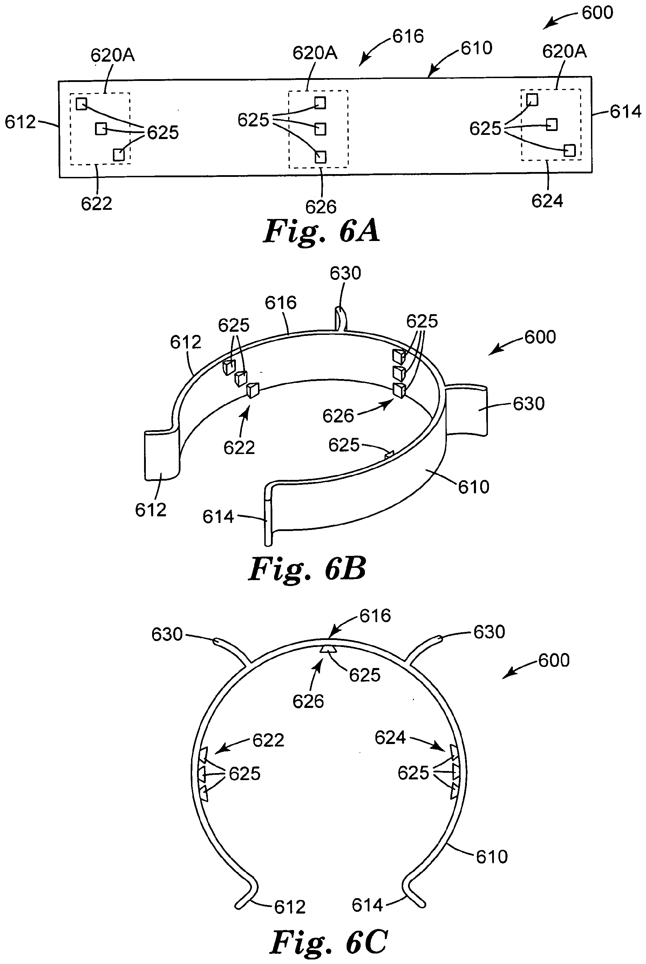

Figure 6A is a flattened view of a hose clamp 600 toward the inner surface of an encircling element; Figure 6B is a perspective view of the hose clamp 600; and Figure 6C is a side view of the hose clamp 600. The hose clamp 600 includes an encircling element 610, an optional grabbing component 630 and an optional engaging component 620A. The encircling element 610 has a first end 612, a second end 614, and a middle portion 616. The engaging component 620A can include one or more sets of engaging elements 625. In one embodiment, the engaging component 620A includes a set of engaging elements 622 disposed proximate to the first end 612 of the encircling element 610. In the example illustrated in 6A, the set of engaging elements 622 includes multiple engaging elements 625 (with three illustrated) disposed in a line, where the engaging elements 625 are disposed in a line slanted from the first end 612. In some embodiments, the engaging component 620A includes a set of engaging elements 624 disposed proximate to the second end 614 of the encircling element 610. In the example illustrated in Figure 6A, the set of engaging elements 624 includes multiple engaging elements 625 disposed in a line, where the engaging elements 625 are disposed in a line slanted from the second end 614. In some embodiments, the engaging component 620A includes a set of engaging elements 626 disposed in the middle portion 616. In some cases, the set of engaging elements 626 includes at least three engaging elements 625 disposed in a line.

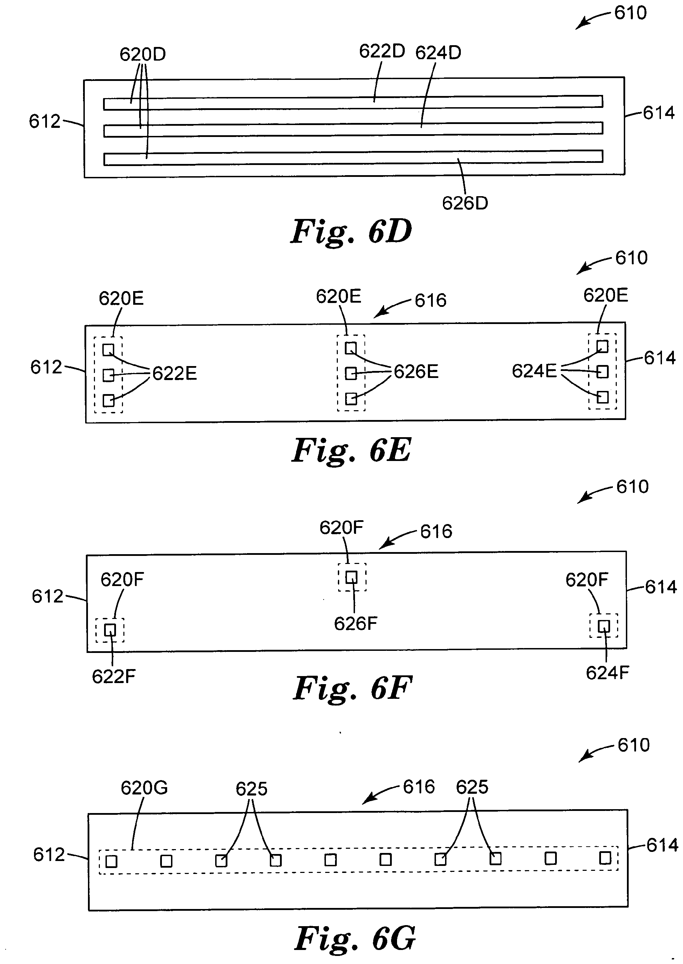

Figures 6D-6G illustrate some example configurations of engaging components. Figure 6D illustrates an engaging component 620D includes three elongated engaging elements 622D, 624D, and 626D that are generally parallel with each other and extend proximate the first end 612 to the second end 614. Figure 6E illustrates an engaging component 620E includes three sets of engaging elements (622E, 624E, and 626E). The set of engaging element 622E is proximate to the first end 612 and is in a line. The set of engaging element 624E is proximate to the second end 614 and is generally in a line. The set of engaging element 626E is proximate to the center portion 616 and is generally parallel to either end.

Figure 6F illustrates an engaging component 620F including three engaging elements (622F, 624F, and 626F). The engaging element 622F is disposed proximate to the first end 612, the engaging element 624F is disposed proximate to the second end 614, and the engaging element 626F is disposed at the center portion 616. The three engaging elements 622F, 624F, and 626F, as illustrated, may be disposed at locations with different distances to the edges of the encircling element 610. Figure 6G illustrates an engaging component 620G including multiple engaging elements 625. In one embodiment, the engaging elements 625 can be disposed discontinuously across the encircling element 610 from the first end 612 to the second end 614.

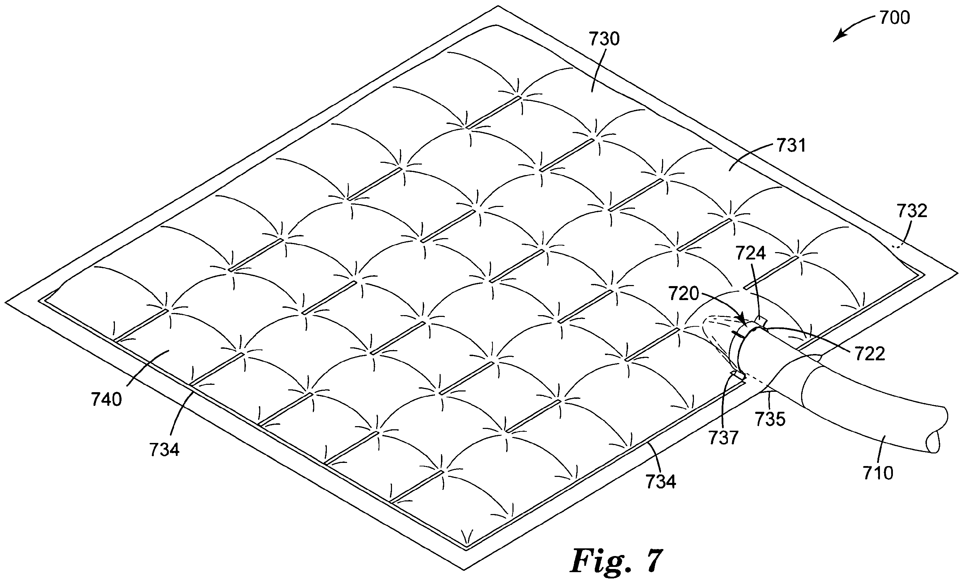

Figure 7 illustrates one embodiment of a system 700 for controlling temperature using a convective device and a hose clamp to maintain hose connection. The system 700 includes a hose 710 configured to connect to an inflation medium source (not illustrated), a hose clamp 720, and a convective device 730. The hose clamp 720 includes an encircling element 722 and an optional grabbing component 724 extending from the encircling element 722. In some embodiments, the convective device 730 includes a flexible first layer 731, a flexible second layer 732 joining the first layer 731 by a seal 734 around a common periphery to form a pneumatic structure 740, and opening 735 into the pneumatic structure 740. The first layer 731 and/or the second layer 732 have an air permeable surface. The hose clamp 720 is configured to maintain generally air-tight connection of the convective device 730 and the hose 710 when an end of the hose 710 is inserted into the opening 735.

The hose clamp 720 can use any one of the hose clamp embodiments described in the present disclosure. In some cases, the encircling element 722 has an inner surface and an opposing outer surface, where the inner surface has a higher friction coefficient than the outer surface. In some cases, the outer surface of the hose 710 has relative high friction coefficient.

In some embodiments, the convective device 730 includes one or two slits 737 along one or both sides of the opening 735, where one or both sides of the hose clamp 720 are configured to go through the slit 737 when the hose clamp 720 is in use. In some cases, the slit 737 is surrounded by a seal (not shown). In some configurations, the slit 737 is at the edge of the convective device 730. In some other configurations, the slit 737 is not at the edge of the convective device 730. In such configurations, the seal surrounding the slit 737 is an enclosed seal. The slit 737 can include, for example, an elongate opening, a line of weakness, perforation, or the like.

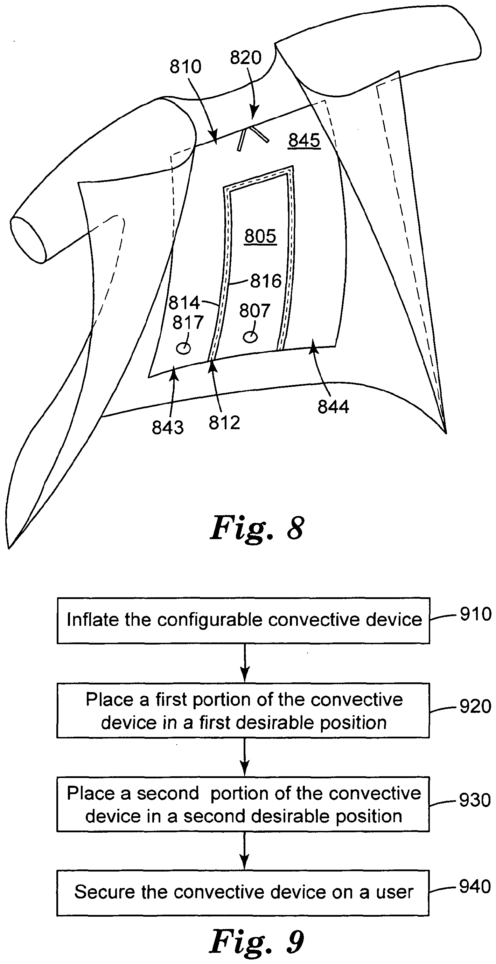

In some cases, one or more convective devices with any convective device embodiments described herein, can be attached to a gown and to be released from the gown. Figure 8 illustrates one embodiment of a gown 800 with a convective device 810 using any convective device embodiments described herein. In some cases, the convective device 810 can be folded and included in a sealed pouch or pocket of the gown 800. In such cases, the convective device 810 can be taken out of the pouch or pocket when it is to be used. In some cases, the convective device 810 can be releasably attached to the gown 800 using an attachment device. An attachment device can use any releasable attachment means, for example, two-sided adhesive, perforated tear-away tabs, hook and loop, snaps, rivets, repositionable adhesives, mechanical reclosable fasteners, or the like. In some configurations, the convective device 810 can be folded separately from the gown 800. For example, the convective device 810 can be stored in a pocket of the gown 800. As another example, the convective device 810 and the gown 800 can dispose together in a kit. In the embodiment illustrated, the gown includes an additional convective device 805. The convective device 810 includes two portions (843, 844) and an inflatable channel 845 connecting the two portions.

In some embodiments, the convective device 810 has two portions (843, 844). The convective device 810 is separated from the convective device 806 by a separation device 812. The separation device 812 includes a seal 814 and a separation element 816. In some cases, the convective device 810 includes an air-guide device 820 disposed in the inflatable channel 845. The convective device 810 includes an opening 817 to connect to an inflation medium source. The convective device 805 includes an opening 807 to connect to an inflation medium source. The separation device 812 and air-guide device 820 can use any embodiments as disclosed herein. In some cases, the convective device 810 can be released from the gown and rearranged such that the two portions 843 and 844 are arranged to a different relative position (e.g., extended approximately along a same line). In such cases, the air-guide device 820 is configured to facilitate forming creases at the edge of the air-guide device when the configurable convective device 800 is inflated to reduce pressure drop of the inflation medium at the bending area.

Figure 9 illustrates an example flow diagram of using a configurable convective device. The configurable convective device can use any one of the configurations described herein. First, inflate the configurable convective device (step 910). As an optional step, if the configurable convective device includes a separation device, use the separated device to partially separate the convective device into two portions. Place a first portion of the convective device in a first desirable position (step 920), for example, by bending the first portion to an angle. Optionally, place a second portion of the convective in a second desirable position (step 930), for example, by bending the first portion to an angle. Secure the convective device on a user(step 940), for example, using the placement device of the convective device such as tie strips or adhesive strips, using a sheet to affix the convective device, or by tucking in a portion of the convective device under a body part of the user.

The present invention should not be considered limited to the particular examples and embodiments described above, as such embodiments are described in detail to facilitate explanation of various aspects of the invention. Rather the present invention should be understood to cover all aspects of the invention, including various modifications, equivalent processes, and alternative devices falling within the scope of the invention as defined by the appended claims.

## Claims

1. A configurable convective device (100, 200, 300A, 300L), comprising:
a flexible first layer (107, 207, 307A, 307L) having air permeable surface;
a flexible second layer joining the first layer by a periphery seal (106, 206, 306A, 306L) around a common periphery to form a pneumatic structure (140, 240, 340A, 340L), the pneumatic structure having a first edge (141, 241, 341A, 341L) and an opposing second edge (142, 242, 342A, 342L), wherein the pneumatic structure comprises a first portion (143, 243, 343A, 343L), a second portion (144, 244, 344A, 344L), and an inflatable channel (145, 245, 345A, 345L) connecting the first portion and the second portion, wherein the first portion (143, 243, 343A, 343L) and the second portion (144, 244, 344A, 344L) define a plane;
at least one opening (130, 230, 330A, 330L) into the pneumatic structure; and
an air-guide device (120, 220, 320A, 320L) disposed proximate to a periphery of the inflatable channel (145, 245, 345A, 345L) connecting the first portion (143, 243, 343A, 343L) and the second portion (144, 244, 344A, 344L), the air-guide device (120, 220, 320, 320L) configured to direct flow of inflation medium by facilitating forming creases at the edge of the air-guide device to reduce kinking effects and air pressure drop when the first portion (143, 243, 343A, 343L) and the second portion (144, 244, 344A, 344L) are bent with respect to each other within the plane, wherein the air-guide device (120, 220, 320A, 320L) comprises two guiding seals (121, 122, 221, 222, 321A, 322A, 321L, 322L), each guiding seal extending from the periphery seal (106, 206, 306A, 306L) proximate to the second edge (142, 242, 342A, 342L) and toward the pneumatic structure (140, 240, 340A, 340L), wherein the two guiding seals (121, 122, 221, 222, 321A, 322A, 321L, 322L) are directed to the first portion (143, 243, 343A, 343L) and the second portion (144, 244, 344A, 344L), respectively, of the pneumatic structure (140, 240, 340A, 340L).

2. The configurable convective device of claim 1, wherein the air-guide device comprises a wavy seal (321C, 322C, 323C, 324C, 321G, 322G, 323G, 324G, 3211, 3221) integrated with the periphery seal.

3. The configurable convective device of claim 1, further comprising:
a separation device (310A, 310C) disposed on the first edge and between the first portion and the second portion, comprising:
an enclosure seal (316A, 312C) extending from the first edge toward the second edge,
a separation element (314A, 314C) within the enclosure seal.

4. The configurable convective device of claim 3, wherein the separation element comprises at least one of a line of weakness, perforation, and slit.

5. The configurable convective device of claim 1, wherein the air-guide device comprises a plurality of small seals (321E, 322E, 323E, 324E) disposed proximate to the periphery seal, wherein a small seal is a seal having a length or diameter less than 5.08 cm (2 inches).

6. The configurable convective device of claim 1, further comprising:
a head warmer (360G) configured to be disposed proximate to a person's neck, wherein the head warmer comprises one or more inflatable sections (361G, 362G) partially separated from the configurable convective device.

## Patentansprüche

1. Konfigurierbare konvektive Vorrichtung (100, 200, 300A, 300L), die aufweist:
eine flexible erste Schicht (107, 207, 307A, 307L), die eine luftdurchlässige Oberfläche aufweist;
eine flexible zweite Schicht, die durch eine Umfangsdichtung (106, 206, 306A, 306L) um einen gemeinsamen Umfang mit der ersten Schicht verbunden ist, um eine pneumatische Struktur (140, 240, 340A, 340L) zu bilden, wobei die pneumatische Struktur eine erste Kante (141, 241, 341A, 341L) und eine gegenüberliegende zweite Kante (142, 242, 342A, 342L) aufweist, wobei die pneumatische Struktur einen ersten Abschnitt (143, 243, 343A, 343L), einen zweiten Abschnitt (144, 244, 344A, 344L) und einen aufblasbaren Kanal (145, 245, 345A, 345L) aufweist, der den ersten Abschnitt und den zweiten Abschnitt verbindet, wobei der erste Abschnitt (143, 243, 343A, 343L) und der zweite Abschnitt (144, 244, 344A, 344L) eine Ebene definieren;
mindestens eine Öffnung (130, 230, 330A, 330L) in die pneumatische Struktur; und
eine Luftleitvorrichtung (120, 220, 320A, 320L), die benachbart zu einem Umfang des aufblasbaren Kanals (145, 245, 345A, 345L) angeordnet ist, der den ersten Abschnitt (143, 243, 343A, 343L) und den zweiten Abschnitt (144, 244, 344A, 344L) verbindet, wobei die Luftleitvorrichtung (120, 220, 320, 320L) konfiguriert ist, um den Fluss von Aufblasmedium zu leiten, indem die Faltenbildung an der Kante der Luftleitvorrichtung ermöglicht wird, um Knickwirkungen und den Luftdruckabfall zu reduzieren, wenn der erste Abschnitt (143, 243, 343A, 343L) und der zweite Abschnitt (144, 244, 344A, 344L) in Bezug zueinander in der Ebene gebogen werden, wobei die Luftleitvorrichtung (120, 220, 320A, 320L) zwei Leitdichtungen (121, 122, 221, 222, 321A, 322A, 321L, 322L) aufweist, wobei sich jede Leitdichtung von der Umfangsdichtung (106, 206, 306A, 306L) benachbart zur zweiten Kante (142, 242, 342A, 342L) und in Richtung der pneumatischen Struktur (140, 240, 340A, 340L) erstreckt, wobei die beiden Leitdichtungen (121, 122, 221, 222, 321A, 322A, 321L, 322L) zum ersten Abschnitt (143, 243, 343A, 343L) bzw. zum zweiten Abschnitt (144, 244, 344A, 344L) der pneumatischen Struktur (140, 240, 340A, 340L) gerichtet sind.

2. Konfigurierbare konvektive Vorrichtung nach Anspruch 1, wobei die Luftleitvorrichtung eine gewellte Dichtung (321C, 322C, 323C, 324C, 321G, 322G, 323G, 324G, 321I, 322I) aufweist, die mit der Umfangsdichtung integriert ist.

3. Konfigurierbare konvektive Vorrichtung nach Anspruch 1, ferner aufweisend:
eine Trennvorrichtung (310A, 310C), die an der ersten Kante und zwischen dem ersten Abschnitt und dem zweiten Abschnitt angeordnet ist, aufweisend:
eine Gehäusedichtung (316A, 312C), die sich von der ersten Kante zur zweiten Kante erstreckt,
ein Trennelement (314A, 314C) innerhalb der Gehäusedichtung.

4. Konfigurierbare konvektive Vorrichtung nach Anspruch 3, wobei das Trennelement mindestens eines von einer Schwächungslinie, einer Perforation und einem Schlitz aufweist.

5. Konfigurierbare konvektive Vorrichtung nach Anspruch 1, wobei die Luftleitvorrichtung eine Mehrzahl an kleinen Dichtungen (321E, 322E, 323E, 324E) aufweist, die benachbart zu der Umfangsdichtung angeordnet sind, wobei eine kleine Dichtung eine Dichtung mit einer Länge oder einem Durchmesser von weniger als 5,08- cm (2 Zoll) ist.

6. Konfigurierbare konvektive Vorrichtung nach Anspruch 1, ferner aufweisend:
einen Kopfwärmer (360G), der so konfiguriert ist, dass er in der Nähe des Halses einer Person angeordnet werden kann, wobei der Kopfwärmer einen oder mehrere aufblasbare Abschnitte (361G, 362G) aufweist, die teilweise von der konfigurierbaren konvektiven Vorrichtung getrennt sind.

## Revendications

1. Dispositif de convection configurable (100, 200, 300A, 300L), comprenant :
une première couche flexible (107, 207, 307A, 307L) ayant une surface perméable à l'air ;
une deuxième couche souple reliant la première couche par un joint périphérique (106, 206, 306A, 306L) autour d'une périphérie commune pour former une structure pneumatique (140, 240, 340A, 340L), la structure pneumatique ayant un premier bord (141, 241, 341A, 341L) et un deuxième bord opposé (142, 242, 342A, 342L), dans lequel la structure pneumatique comprend une première partie (143, 243, 343A, 343A, 343L), une deuxième partie (144, 244, 344A, 344L), et un canal gonflable (145, 245, 345A, 345L) reliant la première partie et la deuxième partie, dans lequel la première partie (143, 243, 343A, 343L) et la deuxième partie (144, 244, 344A, 344L) définissent un plan ;
au moins une ouverture (130, 230, 330A, 330L) dans la structure pneumatique ; et
un dispositif de guidage d'air (120, 220, 320A, 320L) disposé à proximité d'une périphérie du canal gonflable (145, 245, 345A, 345L) reliant la première partie (143, 243, 343A, 343L) et la deuxième partie (144, 244, 344A, 344L), le dispositif de guidage d'air (120, 220, 320, 320L) configuré pour diriger l'écoulement d'un milieu de gonflage en facilitant la formation de pliures au bord du dispositif de guidage d'air pour réduire des effets de vrillage et une chute de pression de l'air lorsque la première partie (143, 243, 343A, 343L) et la deuxième partie (144, 244, 344A, 344L) sont pliées l'une par rapport à l'autre dans le plan, dans lequel le dispositif de guidage d'air (120, 220, 320A 320L) comprend deux joints de guidage (121, 122, 221, 222, 321A, 322A, 321L, 322L), chaque joint de guidage s'étendant à partir du joint périphérique (106, 206, 306A, 306L) à proximité du deuxième bord (142, 242, 342A, 342L) et en direction de la structure pneumatique (140, 240, 340A, 340L), dans lequel les deux joints de guidage (121, 122, 221, 222, 321A, 322A, 321L, 322L) sont dirigés vers la première partie (143, 243, 343A, 343L) et la deuxième partie (144, 244, 344A, 344L), respectivement, de la structure pneumatique (140, 240, 340A, 340L).

2. Dispositif de convection configurable selon la revendication 1, dans lequel le dispositif de guidage d'air comprend un joint ondulé (321C, 322C, 323C, 324C, 321G, 322G, 323G, 324G, 3211, 3221) intégré au joint périphérique.

3. Dispositif de convection configurable selon la revendication 1, comprenant en outre :
un dispositif de séparation (310A, 31 OC) disposé sur le premier bord et entre la première partie et la deuxième partie, comprenant :
un joint d'enceinte (316A, 312C) s'étendant à partir du premier bord vers le deuxième bord,
un élément de séparation (314A, 314C) à l'intérieur du joint d'enceinte.

4. Dispositif de convection configurable selon la revendication 3, dans lequel l'élément de séparation comprend au moins l'une d'une ligne de faiblesse, d'une perforation et d'une fente.

5. Dispositif de convection configurable selon la revendication 1, dans lequel le dispositif de guidage d'air comprend une pluralité de petits joints (321E, 322E, 323E, 324E) disposés à proximité du joint périphérique, dans lequel un petit joint est un joint ayant une longueur ou un diamètre inférieur à 5,08 cm (2 pouces).

6. Dispositif de convection configurable selon la revendication 1, comprenant en outre :
un dispositif de chauffage de tête (360G) configuré pour être disposé à proximité du cou d'une personne, dans lequel le dispositif de chauffage de tête comprend une ou plusieurs sections gonflables (361 G, 362G) partiellement séparées du dispositif de convection configurable.
